Europäisches Patentamt

European Patent Office

Office européen des brevets

(11) Publication number: **0 089 143**
A1

## (12) EUROPEAN PATENT APPLICATION

(21) Application number: 83301117.4

(22) Date of filing: 02.03.83

(51) Int. Cl.³: **A 61 K 49/02**
//G01N31/08

(30) Priority: 16.03.82 GB 8207565

(43) Date of publication of application:
21.09.83 Bulletin 83/38

(84) Designated Contracting States:
DE FR GB

(71) Applicant: AMERSHAM INTERNATIONAL plc
White Lion Road
Amersham Buckinghamshire, HP7 9LL(GB)

(72) Inventor: Nowotnik, David Peter
Amersham International plc White Lion Road
Amersham Buckinghamshire, HP7 9LL(GB)

(74) Representative: Pennant, Pyers et al,
Stevens, Hewlett & Perkins 5 Quality Court Chancery
Lane
London, WC2A 1HZ(GB)

(54) Diagnosis of kidney function.

(57) A novel technetium-99m complex with thiodiglycollic acid, of unknown structure, has renal clearance properties in rats similar to Hippuran $-I-$ 131, and is useful as a gamma camera renography agent. When an aqueous solution of pertechnetate $TcO_4^-$ is reduced with tin metal or $Sn^{++}$ in the presence of thiodiglycollic acid, there results a mixture of two complexes, designated *1 and *2, which can be separated by high performance liquid chromatography. Complex *1 can be converted by heating the mixture into the desired complex *2 which has superior renal clearance.

EP 0 089 143 A1

**0089143**

PP/JR/1694

## DIAGNOSIS OF KIDNEY FUNCTION

The well established nuclear medicine diagnostic technique of renography gives a dynamic representation of the removal of substances from the blood by the kidneys, and their excretion into the urine. A radioactive material which is rapidly excreted from the kidneys is injected into the patient, and its passage through the kidneys is observed with detectors placed over the kidneys (probe renography). The current material of choice for this purpose is sodium iodo-hippurate ("Hippuran") I-131.

Instead of using a single detector over each kidney, it is possible to use a gamma camera. Not only is this much more convenient, especially with children, since accurate positioning is no longer essential, but the results can be stored on tape and the tape replayed in such a way as to accept only the signals from a selected area of the kidney. For this and other reasons, the technique of gamma camera renography is potentially a very powerful one.

Development of the technique has however been held up by the difficulty of finding a suitable radioactive material for injection into the patient. A gamma camera renogram requires much larger quantities of activity than does a simple probe renogram, and, with iodine-131, the radiation dose to the patient (especially one with impaired renal function) would be quite unacceptable. For many years, therefore, workers in the field have attempted to find a suitable Tc-99m complex which could be used in place of Hippuran I-131. This invention results from the discovery of such a substance.

The kidney contains about 1 million nephrons, each comprising a glomerulus and a tubule. The glomerulus acts as a molecular filter, by allowing water from the blood to pass through (this is the source of the urine) together with all small molecules; it will not allow the passage of large protein molecules such as albumin. The tubules re-absorb most of the water from the filtrate back into the blood, and re-absorb essential small molecules such as glucose; they also allow other small molecules to pass from the blood to the urine (tubular secretion).

Of the blood flowing through the kidney, about 20% of the water present passes through the glomerular filter. Thus, if a substance is removed from the blood only by glomerular filtration, only 20% of it will be removed from the blood in a single pass through the kidney. However, if it is removed effectively also by tubular secretion, up to 100% of it may be removed from the blood in a single pass.

To obtain a renogram with good diagnostic properties, as much as possible of the radioactive substance should be removed in a single pass. About 80% of Hippuran is removed in a single pass. Other substances are known which are removed as efficiently as Hippuran, but like Hippuran they cannot be labelled with technetium-99m.

An example of substances which are removed from the blood by glomerular filtration only are metal complexes of diethylene triamine pentacetic acid (DTPA). DTPA is easily labelled with technetium-99m, and the labelled product has been used for gamma camera renograms. But DTPA -Tc-99m, shows a clearance rate only about 20% of that of Hippuran -1-131, and as a result, renograms obtained with DTPA -Tc-99m are flat and of limited diagnostic value. A large number of substances are known which are removed from the blood by glomerular filtration only, and many of these could be labelled with technetium-99m, but renograms obtained using these substances would suffer from the same disadvantages as those obtained from DTPA.

Three necessary features for a gamma camera renography agent are:-

a)   it must be capable of being removed from the blood by tubular secretion as well as by glomerular filtration in the kidney;

b)   it must not accumulate significantly in the kidney or any other organ;   and

c)   it must be capable of being labelled with technetium-99m.

Thiodiglycollic acid (TDG) has the formula:-

$$HOOC - CH_2 - S - CH_2 - COOH$$

The complex of TDG with technetium-99m (TDG-Tc-99m) has been described by Inoue O, Ikeda I and Kurata K in Radioisotopes 1976, 25, 24 - 30.   The authors were looking for agents for renal scanning, that is to say, for static agents which would give a high kidney to background emission ratio.   For this purpose, they required agents which would become localised in the kidney.   Although there is some confusion between TDG and DTG (dithiodiglycollic acid) in the article, their conclusions were that TDG-Tc-99m was excreted rapidly outside the kidney;   and appeared to behave similarly to DTPA-Tc-99m;   and was therefore unsuitable for their purpose.

The use of TDG-Tc-99m for renography forms the subject of our British Patent Application 8112866 filed only 27th April 1981.   TDG-Tc-99m is removed from the blood by tubular secretion as well as by glomerular filtration in the kidneys;   but the clearance rate is not as high as that of Hippuran -I-131.

The present invention results from our discovery that preparation of TDG-Tc-99m can result in a mixture of two complexes of which one shows a higher renal clearance rate than the other. We describe preparation of a mixture of the two complexes and recovery of the separated complexes from the mixture. We describe how one complex may be converted into the other. We give some comparative animal data for the two complexes.

In one aspect the present invention provides a technetium-99m containing material useful as a gamma camera renography agent and characterized by the following properties:-

a) reduction of an aqueous solution of pertechnetate $TcO_4^-$ with a tin reducing agent in the presence of thiodiglycollic acid gives rise to a mixture of two complexes of technetium-99m with thiodiglycollic acid designated *1 and *2, of which complex *2 is the claimed technetium-99m containing material;

b) when the mixture from a) is subjected to high performance liquid chromatography using a 250 x 4.6 mm stainless steel column containing a packing material composed of spherical silica particles with a mean diameter of 5 microns carrying bonded aminopropyl residues, using an aqueous solution of thiodiglycollic acid (0.06M, pH 4.0) as eluent at a flow rate of 1 ml/min, such that the retention times of pertechnetate and complex *1 are about 5 minutes and 10 minutes respectively, the retention time of complex *2 is approximately 12.5 minutes;

c) when the mixture from a) is heated, complex *1 is converted into complex *2; and

d) when injected into rats, complex *2 shows renal clearance properties similar to Hippuran -I-131.

In this statement of invention and in claim 1 we define the material (complex *2) by reference to a method for its preparation. But the invention encompasses the new material per se, irrespective of how it may have been made. If the material is made by reduction of pertechnetate, the choice of reducing agents is not confined to tin reducing agents (stannous tin and tin metal), and other reducing agents may in principle be employed and may be effective to form the claimed material.

As described in our British Patent Application 8112866, TDG-Tc-99m (the mixture) may be prepared by two methods:-

a) To a mixture containing $Sn^{++}$, and TDG is added an aqueous solution of pertechnetate $TcO_4^-$. Improved and more reliable results may be obtained by performing method a) in the presence of cuprous ion and/or nitrate ion.

b) To a mixture containing tin metal, $Cu^{++}$ and TDG is added an aqueous solution of pertechnetate $TcO_4^-$.

When the product of method a) or method b) is subjected to purification by means of high peformance liquid chromatography (HPLC) under suitable conditions, two radioactive components are recovered, herein designated *1 and *2. Typical ratios of *1/*2 are 2.0 - 4.0/1. As indicated below, complex *2 has more advantageous properties for renography than complex *1. It may be possible to adjust the conditions of preparation so as to obtain a higher yield of complex *2.

At ambient temperature complex *2 appears stable over several hours, while complex *1 very slowly breaks down to form largely *2 and Tc-colloid. In one case, on standing overnight, the ratio of *1/*2 decreased from 3/1 to 1/1.

The rate of conversion of *1 to *2 is temperature dependent, being decreased at low ($2^{o}C$) temperatures, and increased at temperatures above ambient. Complex *2 may be obtained from the mixture in yields greater than 90% by heating at $80^{o}C$ for 40 minutes, or $100^{o}C$ for

15 minutes, or 120°C for 5 minutes. Heating the mixture under sterilizing conditions (e.g. 5 minutes at 120°C) provides a convenient way of effecting the desired conversion. Thus, time and temperature of heating may be chosen (having regard to the 6 hour half-life of Tc-99m) to obtain complex *2 in desired yield and purity.

The following Examples illustrate the invention.

### Example 1
### Separation of Complexes *1 and *2 The two Tc-complexes of TDG may be separated by anion exchange HPLC. Two examples of this technique are outlined, below:-

i) The aqueous solution of TDG-Tc-99m resulting from preparation b) above is subjected to HPLC using a 250 x 4.6mm stainless steel column, containing a packing material composed of spherical silica particles with a mean surface area of $200m^2/g$, carrying bonded aminopropyl residues, with an aqueous solution of TDG (0.06M, pH 4.0) as eluent, at a flow rate of 1 ml/min. With this system two Tc-complexes of TDG are observed (denoted *1 and *2), with the retention times of 10 and 12.5 mins, respectively. $TcO_4^-$ has a retention time of 5 minutes. Typical ratios of *1/*2 are 2.0 to 4.0/1.

ii) In a procedure similar to above in which the packing material was composed of irregular shaped silica particles with mean surface area $400m^2/g$ carrying quaternary ammonium residues, and the eluent was 25mM aqueous sodium sulphate, retention times for the two complexes were 6.6(*1) and 8.4(*2) minutes.

. Both methods allow physical separation of the two complexes. HPLC analysis of the separated complexes has demonstrated that both may be obtained with radiochemical purities in excess of 90%.

0089143

## Example 2
### Animal Studies

Solutions of complexes *1 and *2 from Example 1 were injected into rats and the biodistribution pattern noted. The results of these experiments in rats are summarised in Table 1 below, comparing the data on *1 and *2 with earlier data on TDG-Tc-99m, Hippuran -I-131 and DTPA-Tc-99m under identical conditions. In all cases, data from animal experiments was used only when the renal performance appeared "normal", i.e. no unilateral or bilateral renal retention. Data was also rejected in cases of significant retention of activity at the injection site.

|  | Time (min) | DTPA-Tc-99m | Complex *1 | Complex *2 | Hippuran-I-131 | TDG-Tc-99m |
|---|---|---|---|---|---|---|
| **% in bladder of that at 2 hrs.** | 5 |  | 12.0 | 24.9 | 33.4 |  |
|  | 10 | 33.3 | 29.9 | 43.6 | 53.8 | 44.1 |
|  | 15 |  | 45.9 | 66.8 | 63.9 |  |
|  | 20 | 53.9 | 58.1 | 77.4 | 72.4 | 65.4 |
|  | 25 |  | 68.4 | 81.8 | 80.5 |  |
|  | 30 | 67.5 | 70.7 | 85.9 | 85.0 | 76.4 |
|  | 60 | 87.8 | 91.2 | 96.0 | 93.0 | 84.4 |
| **No. of animals** |  | 5 | 4 | 5 | 7 | 8 |
| **Dissection at 2 hr; % of injected dose:** |  |  |  |  |  |  |
| Bladder |  | 96.0 | 95.6 | 98.5 | 93.0 | 94.5 |
| Kidneys |  | 0.96 | 0.44 | 0.25 | 0.2 | 0.54 |
| Liver |  | 0.3 | 0.6 | 0.14 | 0.2 | 1.4 |

Table 1

Complex *1 shows similar renal clearance to DTPA-Tc-99m and *2 is similar to Hippuran -I-131. *2 demonstrates a lower percentage of activity in the bladder over the first ten minutes compared with Hippuran -I-131; this may be due to a difference in renal transit times of the agents. Hippuran -I-131 demonstrates some hepatobiliary excretion: *2 does not.

## Example 3

A sealed, nitrogen filled vial (containing the freeze dried formulation of 20 mg TDG 0.5 mg $CuCl_2$ (pH 4.0) and a small disc of tin metal) was treated with 2 ml of eluate from a technetium generator (8mCi/ml). The vial was allowed to stand at ambient temperature for 20 minutes, then heated in a boiling water bath for 15 minutes, to convert complex *1 to complex *2. On cooling, the solution was subjected to HPLC analysis (under the conditions described in Example 1), which demonstrated the mixture contained 93.6% of *2 and 6.4% of *1.

The solution was filtered through a 0.22 micron membrane filter prior to administration to rats. The results of these animal experiments are shown in Table 2 below.

## Example 4

A sealed nitrogen filled vial (containing the freeze dried formulation of 10 mg TDG, 0.5mg $SnCl_2$, 1 mg NaF at pH 4.0) was treated with 2 ml of eluate from a technetium generator (8 mCi/ml). The vial was allowed to stand at ambient temperature for 20 minutes, then heated in a boiling water bath to convert complex *1 to complex *2. On cooling, the solution was subjected to HPLC analysis (under the conditions described in Example 1), which demonstrated that the mixture contained 94.8% of complex *2 and 3.8% of complex *1.

The solution was filtered through a 0.22 micron membrane filter prior to adminstration to rats.   The results of these animal experiments are shown in Table 2 below.

Heating of the starting mixtures of Examples 3 and 4 was repeated at various temperatures up to $130^{\circ}C$ and for various times.   Satisfactory results were obtained at all temperatures, and it was shown that the rate of conversion of complex *1 to complex *2 is temperature dependent.

Table 2

| ANIMAL STUDIES Time (min) | | Example 3 | | Example 4 | |
|---|---|---|---|---|---|
| | | Rat 1 | Rat 2 | Rat 1 | Rat 2 |
| % in | 5 | 26.5 | 19.5 | 18.1 | 26.1 |
| bladder | 10 | 50.9 | 41.1 | 48.2 | 49.6 |
| of that | 15 | 66.5 | 56.5 | 62.8 | 64.1 |
| at 2 hrs | 20 | 75.7 | 74.4 | 70.4 | 71.7 |
| | 25 | 82.7 | 82.8 | 76.3 | 75.4 |
| | 30 | 86.5 | 86.8 | 83.1 | 82.7 |
| | 60 | 98.6 | 95.3 | 95.2 | 97.7 |
| Dissection at 2 hours;  % of injected dose | | | | | |
| Bladder and urine | | 98.7 | 98.4 | 97.5 | 97.4 |
| Kidneys | | 0.15 | 0.4 | 0.3 | 0.4 |
| Liver | | 0.3 | 0.2 | 0.4 | 0.3 |

## Example 5

A sealed nitrogen filled vial, containing the freeze dried formulation of 20mg TDG, 0.15 mg $SnF_2$, 1 mg NaF and 0.5 mg sodium nitrate was treated with 2 ml of eluate from a technetium generator (6mCi/ml).   The vial was allowed to stand at ambient temperature for 10 minutes, then heated in a boiling water bath for 15 minutes. On cooling, the solution was administered to rats; the results of these animal experiments are shown in Table 3.

Table 3

| | Time (min) | Rat 1 | 2 | Dissection at 2 hrs; % i.d. 1 | 2 |
|---|---|---|---|---|---|
| % in bladder of that at 2 hrs | 5 | 29.1 | 21.1 | | |
| | 10 | 53.9 | 45.9 | Kidneys 0.6 | 0.6 |
| | 15 | 67.2 | 58.6 | Bladder+ | |
| | 20 | 74.2 | 68.8 | Urine 95.2 | 95.0 |
| | 25 | 81.5 | 74.7 | Liver 1.1 | 0.6 |
| | 30 | 84.5 | 79.7 | | |
| | 60 | 95.7 | 89.1 | | |

## C L A I M S

1.    A technetium-99m containing material useful as a gamma camera renography agent and characterized by the following properties:-

a)    reduction of an aqueous solution of pertechnetate $TcO_4^-$ with a tin reducing agent in the presence of thiodiglycollic acid gives rise to a mixture of two complexes of technetium-99m with thiodiglycollic acid designated *1 and *2, of which complex *2 is the claimed technetium-99m containing material;

b)    when the mixture from a) is subjected to high performance liquid chromatography using a 250 x 4.6 mm stainless steel column containing a packing material composed of spherical silica particles with a mean diameter of 5 microns carrying bonded aminopropyl residues, using an aqueous solution of thiodiglycollic acid (0.06M, pH 4.0) as eluent at a flow rate of 1 ml/min, such that the retention times of pertechentate and complex *1 are about 5 minutes and 10 minutes respectively the retention time of complex *2 is approximately 12.5 minutes;

c)    when the mixture from a) is heated, complex *1 is converted into complex *2;  and

d)    when injected into rats, complex *2 shows renal clearance properties similar to Hippuran-I-131.

2.    A method of making a technetiumn-99m containing material useful as a gamma camera renography agent, which method comprises reducing an aqueous solution of pertechnetate $TcO_4^-$ with a tin reducing agent in the presence of thiodiglycollic acid to form a mixture of two complexes of technetium-99m with thiodiglycollic acid designated *1 and *2, and heating the mixture to convert complex *1 into complex *2

3.    A method of making a technetium-99m containing material useful as a gamma camera renography agent, which method comprises reducing an aqueous solution of pertechnetate $TcO_4^-$ with tin reducing agents in the presence of thiodiglycollic acid to form a mixture of two complexes of technetium-99m with thiodiglycollic acid designated *1 and *2, subjecting the mixture to high performance liquid chromatography to separate the two complexes, and recovering complex *2.

4.    A method as claimed in claim 2 or claim 3, wherein the tin reducing agent is $Sn^{++}$ in the presence of $Cu^+$ and/or $NO_3^-$.

5.    A method as claimed in claim 2 or claim 3, wherein the tin reducing agent is tin metal in the presence of $Cu^{++}$.

6.    A technetium-99m containing material as defined in claim 1 for use as a gamma camera renography agent.

## DOCUMENTS CONSIDERED TO BE RELEVANT

EP 83301117.4

| Category | Citation of document with indication, where appropriate, of relevant passages | Relevant to claim | CLASSIFICATION OF THE APPLICATION (Int. Cl. ³) |
|---|---|---|---|
| Y | FR - A1 - 2 322 586 (RESEARCH CORPORATION)<br><br>* Claims 1-9 *<br><br>-- | 1,2 | A 61 K 49/02//<br><br>G 01 N 31/08 |
| Y | GB - A - 1 497 904 (SOLCO BASEL AG)<br><br>* Claims 1,7 *<br><br>-- | 1,2 | |
| Y | GB - A - 1 438 919 (F. HOFFMANN-LA ROCHE & CO.A.G<br><br>* Claims 1-4 *<br><br>---- | 1,2 | |

TECHNICAL FIELDS SEARCHED (Int. Cl. ³)

A 61 B

A 61 K

G 01 N

The present search report has been drawn up for all claims

| Place of search | Date of completion of the search | Examiner |
|---|---|---|
| VIENNA | 08-06-1983 | SCHNASS. |